# EUROPÄISCHE PATENTSCHRIFT

(11) **EP 1 204 627 B1**
(45) Veröffentlichungstag und Bekanntmachung des Hinweises auf die Patenterteilung: **15.11.2006**
(21) Anmeldenummer: 00951499.3
(22) Anmeldetag: 11.08.2000
(51) Int. Cl.: C07C 43/178, C08G 65/26, A61K 8/00, C10M 129/16, A01N 31/04, C11D 1/72

(54) **VERFAHREN ZUR HERSTELLUNG VON VERZWEIGTEN, WEITGEHEND UNGESÄTTIGTEN FETTALKOHOLPOLYGLYCOLETHERN**
PROCESS FOR PRODUCING BRANCHED, LARGELY UNSATURATED FATTY ALCOHOL POLYGLYCOLETHERS
PROCEDE DE PREPARATION D'ETHERS DE POLYGLYCOLS D'ALCOOLS GRAS RAMIFIES ET LARGEMENT INSATURES

(30) Priorität: 20.08.1999 DE 19939538
(43) Veröffentlichungstag der Anmeldung: 15.05.2002
(73) Patentinhaber: Cognis IP Management GmbH, 40589 Düsseldorf (DE)
(72) Erfinder: WESTFECHTEL, Alfred, D-40724 Hilden (DE); HÜBNER, Norbert, D-40764 Langenfeld (DE); BEHLER, Ansgar, D-46240 Bottrop (DE)
(86) Internationale Anmeldenummer: PCT/EP2000/007847
(87) Internationale Veröffentlichungsnummer: WO 2001/014301

(56) Entgegenhaltungen:
- DE-A- 4 335 781
- DE-C- 4 422 858
- US-A- 3 887 624
- H. MÖHRING: "Produkte der Dimerisierung ungesättigter Fettsäuren VII: Kinetische Untersuchung der Mono- und Dimeren, die bei der Dimerisierung von Ölsäure entstehen" FAT SCI. TECHNOL., Bd. 94, Nr. 2, 1992, Seiten 41-46, XP002154120 in der Anmeldung erwähnt

## Beschreibung

### Gebiet der Erfindung

Die Erfindung befindet sich auf dem Gebiet der nichtionischen Tenside und betrifft weitgehend ungesättigte Fettalkoholpolyglycolether, die sich infolge von Verzweigungen in der Kohlenwasserstoffkette gegenüber linearen Homologen durch signifikant verbesserte Eigenschaften auszeichnen, ein Verfahren zu deren Herstellung sowie ihre Verwendung zur Herstellung von oberflächenaktiven Mitteln.

### Stand der Technik

Fettalkoholpolyglycolether, die im wesentlichen durch Ethoxylierung und/oder Propoxylierung der entsprechenden talgbasierten Alkenole erhalten werden, stellen wichtige Rohstoffe für die Herstellung sowohl von kosmetischen Zubereitungen als auch von Wasch-, Spül- und Reinigungsmitteln dar. Die vorteilhaften Eigenschaften dieser Stoffe sind an das Vorhandensein der Doppelbindung im Molekül geknüpft, was gleichzeitig jedoch auch Probleme aufwirft, da die ungesättigten Fettalkoholpolyglycolether leicht der Autoxidation anheimfallen, was mit Verfärbungen und unerwünschten chemischen Veränderungen (z.B. Bildung von Peroxiden und Hydroperoxiden) verbunden ist.

Es ist deshalb klar, daß im Markt der Wunsch nach ungesättigten Fettalkoholpolyglycolether mit verbesserter Oxidationsstabilität oder geeigneten Ersatzstoffen besteht, welche über mindestens gleichwertige anwendungstechnische Eigenschaften verfügen. Als Alternative für ungesättigte Fettalkoholpolyglycolether haben indes bislang nur mehr oder minder reine Isostearylalkoholpolyglycolether zur Verfügung gestanden. Zu deren Herstellung ist es jedoch erforderlich, zunächst Ölsäure zu dimerisieren, die Fraktion monomerer, verzweigter Fettsäuren abzutrennen, zu härten, einer fraktionierten Kristallisation zu unterwerfen, die dabei anfallende flüssige, isostearinsäurereiche Fraktion abzutrennen, mit Methanol zu verestern und die Ester anschließend zu den Alkoholen zu hydrieren, welche dann in die Polyglycolether überführt werden.

Das oben geschilderte Verfahren ist indes durch die zwei Hydrierschritte technisch aufwendig und liefert mit den Isostearylalkoholpolyglycolether Ersatzstoffe, die die ungesättigten Fettalkoholpolyglycolether nur bedingt ersetzen können. Somit hat die Aufgabe der vorliegenden Aufgabe darin bestanden, ungesättigte Fettalkoholpolyglycolether zur Verfügung zu stellen, die sich durch verbesserte anwendungstechnische Eigenschaften, vorzugsweise eine höhere Oxidationsstabilität auszeichnen.

### Beschreibung der Erfindung

Gegenstand der Erfindung ist ein Verfahren zur Herstellung von verzweigten, weitgehend ungesättigten Fettalkoholpolyglycolether, bei dem man
(a) ungesättigte Fettsäuren mit 16 bis 22 Kohlenstoffatomen in an sich bekannter Weise dimerisiert,
(b) die bei der Dimerisierung anfallende Monomerfraktion abtrennt,
(c) die in dieser Fraktion enthaltenen verzweigten, verzweigten ungesättigten Fettsäuren in die entsprechenden Fettsäuremethylester überführt,
(d) die verzweigten, weitgehend ungesättigten Fettsäuremethylester unter Erhalt der Doppelbindungen zu den entsprechenden verzweigten, weitgehend ungesättigten Fettalkoholen hydriert, und diese
(e) in an sich bekannter Weise unter Erhalt der Doppelbindung alkoxyliert.

### Herstellung der Fettalkohole

Die Dimerisierung von Fettsäuren und die Gewinnung von Monomerfettsäuren aus den Dimerisaten ist aus dem Stand der Technik hinreichend bekannt. In diesem Zusammenhang sei beispielsweise auf die

Übersichten von A.Behr et al. **[Fat Sci.Technol. 93, 340 (1991)]** sowie H.Möhring et al. **[ibid. 94, 41 (1992) und 94, 241 (1992)]** verwiesen. Die Abfolge der Schritte (a) bis (d) liefert auf Basis von dimerisierten, vorzugsweise einfach ungesättigten C₁₆- bis C₂₂-Fettsäuren, also Ölsäure, Elaidinsäure, Petroselinsäure, Gadoleinsäure und Erucasäure sowie deren Gemischen verzweigte, weitgehend ungesättigte Fettalkohole im Iodzahlbereich von 45 bis 85. Für eine Reihe von Anwendungen ist dies zweifellos völlig ausreichend, werden jedoch Fettstoffe benötigt, die einen höheren Gehalt an ungesättigten Verbindungen aufweisen, empfiehlt es sich, daß man die bei der Dimerisierung anfallende Monomerfraktion zunächst einer fraktionierten Kristallisation unterwirft und die dabei anfallende flüssige Phase gegebenenfalls nach Destillation der Veresterung unterwirft. Die dabei anfallende Fettsäure und deren Methylester stellen eine schon ziemliche reine Isoölsäure bzw. einen Isoölsäuremethylester dar, die eine lodzahl im Bereich 75 bis 95 aufweisen. In jedem Fall ist es ratsam, die Methylester und/oder die Fettalkohole einer Destillation und/oder fraktionierten Kristallisation ("Winterisierung") zu unterwerfen. Die Veresterung der Fettsäuren mit Methanol erfolgt nach den Verfahren des Stands der Technik und dient dazu Methylester zu erzeugen, die sich vergleichsweise leicht hydrieren lassen. Anstelle der Methylester können selbstverständlich auch andere Niedrigalkylester, wie z.B. Ethyl-, Propyl- oder Butylester erzeugt und dann hydriert werden, die Auswahl des Alkohols ist an sich unkritisch und richtet sich ausschließlich nach wirtschaftlichen Kriterien und Verfügbarkeit. Anstelle der Methyl- bzw. Niedrigalkyfester ist es grundsätzlich auch möglich, die Fettsäuren direkt zu hydrieren, allerdings werden für diesen Zweck dann spezielle Katalysatoren benötigt, die mit den Säuren keine Salze bilden; zudem muß das Reaktormaterial korrosionssicher sein. Auch die Hydrierung der ungesättigten Methylester zu den entsprechenden Alkoholen kann in an sich bekannter Weise erfolgen. Entsprechende Verfahren und Katalysatoren, insbesondere solche auf Basis von Kupfer und Zink, sind beispielsweise den folgenden Druckschriften zu entnehmen: **DE 4335781 C1, EP 0602108 B1, US 3193586** und **US 3729520** (Henkel); auf den Inhalt dieser Schriften wird ausdrücklich Bezug genommen.

### Alkoxylierung

Die zuvor erhaltenen verzweigten, weitgehend ungesättigten Fettalkohole werden anschließend in an sich bekannter Weise, unter Erhalt der Doppelbindung, alkoxyliert, d.h. an die Hydroxylgruppe Ethylenoxid, Propylenoxid oder deren Gemische in Random- oder Blockverteilung angelagert.

Vorzugsweise werden durchschnittlich 1 bis 50, insbesondere 5 bis 15 Mol Ethylenoxid und/oder 1 bis 5 Mol Propylenoxid addiert. Die Alkoxylierung erfolgt nach den Verfahren des Stands der Technik, also üblicherweise in Gegenwart alkalischer homogener oder heterogener Katalysatoren, wie z.B. Natriummethylat, Kalium-tert.butylat oder calcinierter bzw. mit Fettsäuren hydrophobierter Hydrotalcit. Dem entsprechend können die Alkoxylate auch eine konventionell breite oder eingeengte Homologenverteilung aufweisen.

### Gewerbliche Anwendbarkeit

Die neuen verzweigten, weitgehend ungesättigten Fettalkoholpolyglycolether zeichnen sich durch besondere Oxidationsstabilität aus und eignen sich daher zur Herstellung von oberflächenaktiven Mitteln, vorzugsweise Wasch-, Spül-, Reinigungs- und Avivagemitteln sowie kosmetischen und/oder pharmazeutischen Zubereitungen, in denen sie in Mengen von 1 bis 50, vorzugsweise 5 bis 35 und insbesondere 10 bis 25 Gew.-% enthalten sein können.

### Wasch-, Spül-, Reinigungs- und Avivagemittel

Die erfindungsgemäßen verzweigten, weitgehend ungesättigten Fettalkoholpolyglycolether als Rohstoffe für die Herstellung von Wasch-, Spül-, Reinigungs- oder Avivagemitteln ("Softener") dienen, liegen in flüssiger Form vor. Flüssige Zubereitungen, die Polyglycolether enthalten, können einen nicht wäßrigen Anteil im Bereich von 5 bis 50 und vorzugsweise 15 bis 35 Gew.-% aufweisen. Im einfachsten Fall handelt es sich um wäßrige Lösungen der genannten Mischungen. Bei den Flüssigwaschmitteln kann es sich aber auch um im wesentlichen wasserfreie Mittel handeln. Dabei bedeutet "im wesentlichen wasserfrei" im Rahmen dieser Erfindung, daß das Mittel vorzugsweise kein freies, nicht als Kristallwasser oder in vergleichbarer Form gebundenes Wasser enthält. In einigen Fällen sind geringe Menge an freiem Wasser tolerierbar, insbesondere in Mengen bis zu 5 Gew.-%. Die im Detergensbereich eingesetzten Mittel können weitere typische Inhaltsstoffe, wie beispielsweise Builder, Bleichmittel, Bleichaktivatoren, Lösungsmittel, Waschkraftverstärker, Enzyme, Enzymstabilisatoren, Viskositätsregulatoren, Vergrauungsinhibitoren, optische Aufheller, Soil repellants, Schauminhibitoren, anorganische Salze sowie Duft- und Farbstoffe enthalten.

Geeignete **flüssige Builder** sind Ethylendiamintetraessigsäure, Nitrilotriessigsäure, Citronensäure sowie anorganische Phosphonsäuren, wie z.B. die neutral reagierenden Natriumsalze von 1-Hydroxyethan-1,1,-diphosphonat, die in Mengen von 0,5 bis 5, vorzugsweise 1 bis 2 Gew.-% zugegen sein können.

Als **feste Builder** wird insbesondere feinkristalliner, synthetisches und gebundenes Wasser enthaltender Zeolith wie Zeolith NaA in Waschmittelqualität eingesetzt. Geeignet sind jedoch auch Zeolith NaX sowie Mischungen aus NaA und NaX. Der Zeolith kann als sprühgetrocknetes Pulver oder auch als ungetrocknete, von ihrer Herstellung noch feuchte, stabilisierte Suspension zum Einsatz kommen. Für den Fall, daß der Zeolith als Suspension eingesetzt wird, kann diese geringe Zusätze an nichtionischen Tensiden als Stabilisatoren enthalten, beispielsweise 1 bis 3 Gew.-%, bezogen auf Zeolith, an ethoxylierten C₁₂-C₁₈-Fettalkoholen mit 2 bis 5 Ethylenoxidgruppen oder ethoxylierte Isotridecanole. Geeignete Zeolithe weisen eine mittlere Teilchengröße von weniger als 10 µm (Volumenverteilung; Meßmethode: Coulter Counter) auf und enthalten vorzugsweise 18 bis 22, insbesondere 20 bis 22 Gew.-% an gebundenem Wasser. Geeignete Substitute bzw. Teilsubstitute für Zeolithe sind kristalline, schichtförmige Natriumsilicate der allgemeinen Formel NaMSiₓO₂ₓ₊₁·yH₂O, wobei M Natrium oder Wasserstoff bedeutet, x eine Zahl von 1,9 bis 4 und y eine Zahl von 0 bis 20 ist und bevorzugte Werte für x 2, 3 oder 4 sind. Derartige kristalline Schichtsilicate werden beispielsweise in der europäischen Patentanmeldung **EP 0164514 A** beschrieben. Bevorzugte kristalline Schichtsilicate sind solche, in denen M in der allgemeinen Formel für Natrium steht und x die Werte 2 oder 3 annimmt. Insbesondere sind sowohl β- als auch γ-Natriumdisilicate Na₂Si₂O₅·yH₂O bevorzugt, wobei β-Natriumdisilicat beispielsweise nach dem Verfahren erhalten werden kann, das in der internationalen Patentanmeldung **WO 91/08171** beschrieben ist. Pulverwaschmittel auf Basis der erfindungsgemäßen verzweigtem weitgehend verzweigten Fettalkoholethersulfate enthalten als feste Builder vorzugsweise 10 bis 60 Gew.-% Zeolith und/oder kristalline Schichtsilicate, wobei Mischungen von Zeolith und kristallinen Schichtsilicaten in einem beliebigen Verhältnis besonders vorteilhaft sein können. Insbesondere ist es bevorzugt, daß die Mittel 20 bis 50 Gew.-% Zeolith und/oder kristalline Schichtsilicate enthalten. Besonders bevorzugte Mittel enthalten bis 40 Gew.-% Zeolith und insbesondere bis 35 Gew.-% Zeolith, jeweils bezogen auf wasserfreie Aktivsubstanz. Weitere geeignete Inhaltsstoffe der Mittel sind wasserlösliche amorphe Silicate; vorzugsweise werden sie in Kombination mit Zeolith und/oder kristallinen Schichtsilicaten eingesetzt. Insbesondere bevorzugt sind dabei Mittel, welche vor allem Natriumsilicat mit einem molaren Verhältnis (Modul) Na₂O : SiO₂ von 1:1 bis 1:4,5, vorzugsweise von 1:2 bis 1:3,5, enthalten. Der Gehalt der Mittel an amorphen Natriumsilicaten beträgt dabei vorzugsweise bis 15 Gew.-% und vorzugsweise zwischen 2 und 8 Gew.-%. Auch Phosphate wie Tripolyphosphate, Pyrophosphate und Orthophosphate können in geringen Mengen in den Mitteln enthalten sein. Vorzugsweise beträgt der Gehalt der Phosphate in den Mitteln bis 15 Gew.-%, jedoch insbesondere 0 bis 10 Gew.-%. Außerdem können die Mittel auch zusätzlich Schichtsilicate natürlichen und synthetischen Ursprungs enthalten. Derartige Schichtsilicate sind beispielsweise aus den Patentanmeldungen **DE 2334899 B, EP 0026529 A** und **DE 3526405 A** bekannt. Ihre Verwendbarkeit ist nicht auf eine spezielle Zusammensetzung bzw. Strukturformel beschränkt. Bevorzugt sind hier jedoch Smectite, insbesondere Bentonite. Geeignete Schichtsilicate, die zur Gruppe der mit Wasser quellfähigen Smectite zählen, sind z.B. solche der allgemeinen Formeln

(OH)₄Si_{8-y}Al_{y}(MgₓAl₄₋ₓ)O₂₀ Montmorrilonit

(OH)₄Si_{8-y}Al_{y}(Mg_{6-z}Li_{z})O₂₀ Hectorit

(OH)₄Si_{8-y}Al_{y}(Mg_{6-z} Al_{z})O₂₀ Saponit

mit x = 0 bis 4, y = 0 bis 2, z = 0 bis 6. Zusätzlich kann in das Kristallgitter der Schichtsilicate gemäß den vorstehenden Formeln geringe Mengen an Eisen eingebaut sein. Ferner können die Schichtsilicate aufgrund ihrer ionenaustauschenden Eigenschaften Wasserstoff-, Alkali-, Erdalkaliionen, insbesondere Na+ und Ca²⁺ enthalten. Die Hydratwassermenge liegt meist im Bereich von 8 bis 20 Gew.-% und ist vom Quellzustand bzw. von der Art der Bearbeitung abhängig. Brauchbare Schichtsilicate sind beispielsweise aus **US 3,966,629, US 4,062,647, EP 0026529 A** und **EP 0028432 A** bekannt. Vorzugsweise werden Schichtsilicate verwendet, die aufgrund einer Alkalibehandlung weitgehend frei von Calciumionen und stark färbenden Eisenionen sind. Brauchbare organische Gerüstsubstanzen sind beispielsweise die bevorzugt in Form ihrer Natriumsalze eingesetzten Polycarbonsäuren, wie Citronensäure, Adipinsäure, Bernsteinsäure, Glutarsäure, Weinsäure, Zuckersäuren, Aminocarbonsäuren, Nitrilotriessigsäure (NTA), sofern ein derartiger Einsatz aus ökologischen Gründen nicht zu beanstanden ist, sowie Mischungen aus diesen. Bevorzugte Salze sind die Salze der Polycarbonsäuren wie Citronensäure, Adipinsäure, Bernsteinsäure, Glutarsäure, Weinsäure, Zuckersäuren und Mischungen aus diesen. Geeignete polymere Polycarboxylate sind beispielsweise die Natriumsalze der Polyacrylsäure oder der Polymethacrylsäure, beispielsweise solche mit einer relativen Molekülmasse von 800 bis 150000 (auf Säure bezogen). Geeignete copolymere Polycarboxylate sind insbesondere solche der Acrylsäure mit Methacrylsäure und der Acrylsäure oder Methacrylsäure mit Maleinsäure. Als besonders geeignet haben sich Copolymere der Acrylsäure mit Maleinsäure erwiesen, die 50 bis 90 Gew.-% Acrylsäure und 50 bis 10 Gew.-% Maleinsäure enthalten. Ihre relative Molekülmasse, bezogen auf freie Säuren, beträgt im allgemeinen 5000 bis 200000, vorzugsweise 10000 bis 120000 und insbesondere 50000 bis 100000. Der Einsatz polymerer Polycarboxylate ist nicht zwingend erforderlich. Falls jedoch polymere Polycarboxylate eingesetzt werden, so sind Mittel bevorzugt, welche biologisch abbaubare Polymere, beispielsweise Terpolymere, die als Monomere Acrylsäure und Maleinsäure bzw. deren Salze sowie Vinylalkohol bzw. Vinylalkohol-Derivate oder die als Monomere Acrylsäure und 2-Alkylallylsulfonsäure bzw. deren Salze sowie Zuckerderivate enthalten. Insbesondere sind Terpolymere bevorzugt, die nach der Lehre der deutschen Patentanmeldungen **DE 4221381 A** und **DE 4300772 A** erhalten werden. Weitere geeignete Buildersubstanzen sind Polyacetale, welche durch Umsetzung von Dialdehyden mit Polyolcarbonsäuren, welche 5 bis 7 Kohlenstoffatome und mindestens 3 Hydroxylgruppen aufweisen, beispielsweise wie in der europäischen Patentanmeldung **EP 0280223 A** beschrieben erhalten werden können. Bevorzugte Polyacetale werden aus Dialdehyden wie Glyoxal, Glutaraldehyd, Terephthalaldehyd sowie deren Gemischen und aus Polyolcarbonsäuren wie Gluconsäure und/oder Glucoheptonsäure erhalten.

Unter den als Bleichmittel dienenden, in Wasser Wasserstoffperoxid liefernden Verbindungen haben das Natriumperborat-Tetrahydrat und das Natriumperborat-Monohydrat eine besondere Bedeutung. Weitere Bleichmittel sind beispielsweise Peroxycarbonat, Citratperhydrate sowie Salze der Persäuren, wie Perbenzoate, Peroxyphthalate oder Diperoxydodecandisäure. Sie werden üblicherweise in Mengen von 8 bis 25 Gew.-% eingesetzt. Bevorzugt ist der Einsatz von Natriumperborat-Monohydrat in Mengen von 10 bis 20 Gew.-% und insbesondere von 10 bis 15 Gew.-%. Durch seine Fähigkeit, unter Ausbildung des Tetrahydrats freies Wasser binden zu können, trägt es zur Erhöhung der Stabilität des Mittels bei.

Um beim Waschen bei Temperaturen von 60°C und darunter eine verbesserte Bleichwirkung zu erreichen, können **Bleichaktivatoren** in die Präparate eingearbeitet werden. Beispiele hierfür sind mit Wasserstoffperoxid organische Persäuren bildende N-Acyl- bzw. O-Acyl-Verbindungen, vorzugsweise N,N'-tetraacylierte Diamine, ferner Carbonsäureanhydride und Ester von Polyolen wie Glucosepentaacetat. Der Gehalt der bleichmittelhaltigen Mittel an Bleichaktivatoren liegt in dem üblichen Bereich, vorzugsweise zwischen 1 und 10 Gew.-% und insbesondere zwischen 3 und 8 Gew.-%. Besonders bevorzugte Bleichaktivatoren sind N,N,N',N'-Tetraacetylethylendiamin und 1,5-Diacetyl-2,4-dioxo-hexahydro-1,3,5-triazin.

Als organische **Lösungsmittel** kommen beispielsweise mono- und/oder polyfunktionelle Alkohole mit 1 bis 6 Kohlenstoffatomen, vorzugsweise mit 1 bis 4 Kohlenstoffatomen in Frage. Bevorzugte Alkohole sind Ethanol, 1,2-Propandiol, Glycerin sowie deren Gemische. Die Mittel enthalten vorzugsweise 2 bis 20 Gew.-% und insbesondere 5 bis 15 Gew.-% Ethanol oder ein beliebiges Gemisch aus Ethanol und 1,2-Propandiol oder insbesondere aus Ethanol und Glycerin. Ebenso ist es möglich, daß die Zubereitungen entweder zusätzlich zu den mono- und/oder polyfunktionellen Alkoholen mit 1 bis 6 Kohlenstoffatomen oder allein Polyethylenglykol mit einer relativen Molekülmasse zwischen 200 und 2000, vorzugsweise bis 600 in Mengen von 2 bis 17 Gew.-% enthalten. Als Hydrotrope können beispielsweise Toluolsulfonat, Xylolsulfonat, Cumolsulfonat oder deren Mischungen eingesetzt werden.

Als Enzyme kommen solche aus der Klasse der Proteasen, Lipasen, Amylasen, Cellulasen bzw. deren Gemische in Frage. Besonders gut geeignet sind aus Bakterienstämmen oder Pilzen, wie Bacillus subtilis, Bacillus licheniformis und Streptomyces griseus gewonnene enzymatische Wirkstoffe. Vorzugsweise werden Proteasen vom Subtilisin-Typ und insbesondere Proteasen, die aus Bacillus lentus gewonnen werden, eingesetzt. Ihr Anteil kann etwa 0,2 bis etwa 2 Gew.-% betragen. Die Enzyme können an Trägerstoffen adsorbiert und/oder in Hüllsubstanzen eingebettet sein, um sie gegen vorzeitige Zersetzung zu schützen. Zusätzlich zu den mono- und polyfunktionellen Alkoholen und den Phosphonaten können die Mittel weitere Enzymstabilisatoren enthalten. Beispielsweise können 0,5 bis 1 Gew.-% Natriumformiat eingesetzt werden. Möglich ist auch der Einsatz von Proteasen, die mit löslichen Calciumsalzen und einem Calciumgehalt von vorzugsweise etwa 1,2-Gew.-%, bezogen auf das Enzym, stabilisiert sind. Besonders vorteilhaft ist jedoch der Einsatz von Borverbindungen, beispielsweise von Borsäure, Boroxid, Borax und anderen Alkalimetallboraten wie den Salzen der Orthoborsäure (H₃BO₃), der Metaborsäure (HBO₂) und der Pyroborsäure (Tetraborsäure H₂B4O₇).

Als **Viskositätsregulatoren** können beispielsweise gehärtetes Rizinusöl, Salze von langkettigen Fettsäuren, die vorzugsweise in Mengen von 0 bis 5 Gew.-% und insbesondere in Mengen von 0,5 bis 2 Gew.-%, beispielsweise Natrium-, Kalium-, Aluminium-, Magnesium- und Titanstearate oder die Natrium- und/oder Kaliumsalze der Behensäure, sowie weitere polymere Verbindungen eingesetzt werden. Zu den letzteren gehören bevorzugt Polyvinylpyrrolidon, Urethane und die Salze polymerer Polycarboxylate, beispielsweise homopolymerer oder copolymerer Polyacrylate, Polymethacrylate und insbesondere Copolymere der Acrylsäure mit Maleinsäure, vorzugsweise solche aus 50 % bis 10 % Maleinsäure. Die relative Molekülmasse der Homopolymeren liegt im allgemeinen zwischen 1000 und 100000, die der Copolymeren zwischen 2000 und 200000, vorzugsweise zwischen 50000 bis 120000, bezogen auf die freie Säure. Insbesondere sind auch wasserlösliche Polyacrylate geeignet, die beispielsweise mit etwa 1 % eines Polyallylethers der Sucrose quervernetzt sind und die eine relative Molekülmasse oberhalb einer Million besitzen. Beispiele hierfür sind die unter dem Namen Carbopol^{®} 940 und 941 erhältlichen Polymere mit verdickender Wirkung. Die quervernetzten Polyacrylate werden vorzugsweise in Mengen nicht über 1 Gew.-%, vorzugsweise in Mengen von 0,2 bis 0,7 Gew.-% eingesetzt. Die Mittel können zusätzlich etwa 5 bis 20 Gew.-% eines partiell veresterten Copolymerisats enthalten, wie es in der europäischen Patentanmeldung **EP 0367049 A** beschrieben ist. Diese partiell veresterten Polymere werden durch Copolymerisation von (a) mindestens einem C₄-C₂₈-Olefin oder Mischungen aus mindestens einem C₄-C₂₈-Olefin mit bis zu 20 Mol% C₁-C₂₈-Alkylvinylethern und (b) ethylenisch ungesättigten Dicarbonsäureanhydriden mit 4 bis 8 Kohlenstoffatomen im Molverhältnis 1:1 zu Copolymerisaten mit K-Werten von 6 bis 100 und anschließende partielle Veresterung der Copolymerisate mit Umsetzungsprodukten wie C₁-C₁₃-Alkoholen, C₈-C₂₂-Fettsäuren, C₁-C₁₂-Alkylphenolen, sekundären C₂-C₃₀-Aminen oder deren Mischungen mit mindestens einem C₂-C₄-Alkylenoxid oder Tetrahydrofuran sowie Hydrolyse der Anhydridgruppen der Copolymerisate zu Carboxylgruppen erhalten, wobei die partielle Veresterung der Copolymerisate soweit geführt wird, daß 5 bis 50 % der Carboxylgruppen der Copolymerisate verestert sind. Bevorzugte Copolymerisate enthalten als ethylenisch ungesättigtes Dicarbonsäureanhydrid Maleinsäureanhydrid. Die partiell veresterten Copolymerisate können entweder in Form der freien Säure oder vorzugsweise in partiell oder vollständig neutralisierter Form vorliegen. Vorteilhafterweise werden die Copolymerisate in Form einer wäßrigen Lösung, insbesondere in Form einer 40 bis 50 Gew.-%igen Lösung eingesetzt. Die Copolymerisate leisten nicht nur einen Beitrag zur Primär- und Sekundärwaschleistung des flüssigen Wasch- und Reinigungsmittels, sondern bewirken auch eine gewünschte Viskositätsemiedrigung der konzentrierten flüssigen Waschmittel. Durch den Einsatz dieser partiell veresterten Copolymerisate werden konzentrierte wäßrige Flüssigwaschmittel erhalten, die unter dem alleinigen Einfluß der Schwerkraft und ohne Einwirkung sonstiger Scherkräfte fließfähig sind. Vorzugsweise beinhalten die konzentrierten wäßrigen Flüssigwaschmittel partiell veresterte Copolymerisate in Mengen von 5 bis 15 Gew.-% und insbesondere in Mengen von 8 bis 12 Gew.-%.

**Vergrauungsinhibitoren** haben die Aufgabe, den von der Faser abgelösten Schmutz in der Flotte suspendiert zu halten und so das Vergrauen zu verhindern. Hierzu sind wasserlösliche Kolloide meist organischer Natur geeignet, beispielsweise die wasserlöslichen Salze polymerer Carbonsäuren, Leim, Gelatine, Salze von Ethercarbonsäuren oder Ethersulfonsäuren der Stärke oder der Cellulose oder Salze von sauren Schwefelsäureestern der Cellulose oder der Stärke. Auch wasserlösliche, saure Gruppen enthaltende Polyamide sind für diesen Zweck geeignet. Weiterhin lassen sich lösliche Stärkepräparate und andere als die obengenannten Stärkeprodukte verwenden, z.B. abgebaute Stärke, Aldehydstärken usw.. Auch Polyvinylpyrrolidon ist brauchbar. Bevorzugt werden jedoch Celluloseether, wie Carboxymethylcellulose, Methylcellulose, Hydroxyalkylcellulose und Mischether, wie Methylhydroxyethylcellulose, Methylhydroxypropylcellulose, Methylcarboxymethylcellulose und deren Gemische sowie Polyvinylpyrrolidon, beispielsweise in Mengen von 0,1 bis 5 Gew.-%, bezogen auf die Mittel.

Die Mittel können als optische **Aufheller** Derivate der Diaminostilbendisulfonsäure bzw. deren Alkalimetallsalze enthalten. Geeignet sind z.B. Salze der 4,4'-Bis(2-anilino-4-morpholino-1,3,5-triazinyl-6-amino)stilben-2,2'-disulfonsäure oder gleichartig aufgebaute Verbindungen, die anstelle der MorpholinoGruppe eine Diethanolaminogruppe, eine Methylaminogruppe, eine Anilinogruppe oder eine 2-Methoxyethylaminogruppe tragen. Weiterhin können Aufheller vom Typ der substituierten Diphenylstyryle anwesend sein, z.B. die Alkalisalze des 4,4'-Bis(2-sulfostyryl)-diphenyls, 4,4'-Bis(4-chlor-3-sulfostyryl)-diphenyls, oder 4-(4-Chlorstyryl)-4'-(2-sulfostyryl)-diphenyls. Auch Gemische der vorgenannten Aufheller können verwendet werden. Einheitlich weiße Granulate werden erhalten, wenn die Mittel außer den üblichen Aufhellern in üblichen Mengen, beispielsweise zwischen 0,1 und 0,5 Gew.-%, vorzugsweise zwischen 0,1 und 0,3 Gew.-%, auch geringe Mengen, beispielsweise 10⁻⁶ bis 10⁻³ Gew.-%, vorzugsweise um 10⁻⁵ Gew.-%, eines blauen Farbstoffs enthalten. Ein besonders bevorzugter Farbstoff ist Tinolux^{®} (Handelsprodukt der Ciba-Geigy).

Als schmutzabweisenden Polymere ("**soil repellants**") kommen solche Stoffe in Frage, die vorzugsweise Ethylenterephthalat- und/oder Polyethylenglycolterephthalatgruppen enthalten, wobei das Molverhältnis Ethylenterephthalat zu Polyethylenglycolterephthalat im Bereich von 50 : 50 bis 90 : 10 liegen kann. Das Molekulargewicht der verknüpfenden Polyethylenglycoleinheiten liegt insbesondere im Bereich von 750 bis 5000, d.h., der Ethoxylierungsgrad der Polyethylenglycolgruppenhaltigen Polymere kann ca. 15 bis 100 betragen. Die Polymeren zeichnen sich durch ein durchschnittliches Molekulargewicht von etwa 5000 bis 200.000 aus und können eine Block-, vorzugsweise aber eine Random-Struktur aufweisen. Bevorzugte Polymere sind solche mit Molverhältnissen Ethylenterephthalat/Polyethylenglycolterephthalat von etwa 65 : 35 bis etwa 90 : 10, vorzugsweise von etwa 70 : 30 bis 80 : 20. Weiterhin bevorzugt sind solche Polymeren, die verknüpfende Polyethylenglycoleinheiten mit einem Molekulargewicht von 750 bis 5000, vorzugsweise von 1000 bis etwa 3000 und ein Molekulargewicht des Polymeren von etwa 10.000 bis etwa 50.000 aufweisen. Beispiele für handelsübliche Polymere sind die Produkte Milease^{®} T (ICI) oder Repelotex^{®} SRP 3 (Rhöne-Poulenc).
Beim Einsatz in maschinellen Waschverfahren kann es von Vorteil sein, den Mitteln übliche Schauminhibitoren zuzusetzen. Hierfür eignen sich beispielsweise Seifen natürlicher oder synthetischer Herkunft, die einen hohen Anteil an C₁₈-C₂₄-Fettsäuren aufweisen. Geeignete nichttensidartige Schauminhibitoren sind beispielsweise Organopolysiloxane und deren Gemische mit mikrofeiner, gegebenenfalls silanierter Kieselsäure sowie Paraffine, Wachse, Mikrokristallinwachse und deren Gemische mit silanierter Kieselsäure oder Bistearylethylendiamid. Mit Vorteilen werden auch Gemische aus verschiedenen Schauminhibitoren verwendet, z.B. solche aus Silikonen, Paraffinen oder Wachsen. Vorzugsweise sind die Schauminhibitoren, insbesondere silikon- oder paraffinhaltige Schauminhibitoren, an eine granulare, in Wasser lösliche bzw. dispergierbare Trägersubstanz gebunden. Insbesondere sind dabei Mischungen aus Paraffinen und Bistearylethylendiamiden bevorzugt.

Der **pH-Wert** flüssiger, insbesondere auch flüssig-konzentrierter Mittel beträgt im allgemeinen 7 bis 10,5, vorzugsweise 7 bis 9,5 und insbesondere 7 bis 8,5. Die Einstellung höherer pH-Werte, beispielsweise oberhalb von 9, kann durch den Einsatz geringer Mengen an Natronlauge oder an alkalischen Salzen wie Natriumcarbonat oder Natriumsilicat erfolgen. Die flüssigen Zubereitungen weisen im allgemeinen Viskositäten zwischen 150 und 10000 mPas (Brookfield-Viskosimeter, Spindel 1, 20 Umdrehungen pro Minute, 20°C) auf. Dabei sind bei den im wesentlichen wasserfreien Mitteln Viskositäten zwischen 150 und 5000 mPas bevorzugt. Die Viskosität dieser wäßrigen Mittel liegt vorzugsweise unter 2000 mPas und liegt insbesondere zwischen 150 und 1000 mPas.

### Herstellung fester Zubereitungen

Das Schüttgewicht der festen Zubereitungen beträgt im allgemeinen 300 bis 1200 g/l, insbesondere 500 bis 1100 g/l. Ihre Herstellung kann nach jedem der bekannten Verfahren wie Mischen, Sprühtrocknung, Granulieren und Extrudieren erfolgen. Geeignet sind insbesondere solche Verfahren, in denen mehrere Teilkomponenten, beispielsweise sprühgetrocknete Komponenten und granulierte und/oder extrudierte Komponenten miteinander vermischt werden. Dabei ist es auch möglich, daß sprühgetrocknete oder granulierte Komponenten nachträglich in der Aufbereitung beispielsweise mit nichtionischen Tensiden, insbesondere ethoxylierten Fettalkoholen, nach den üblichen Verfahren beaufschlagt werden. Insbesondere in Granululations- und Extrusionsverfahren ist es bevorzugt, die gegebenenfalls vorhandenen Aniontenside in Form eines sprühgetrockneten, granulierten oder extrudierten Compounds entweder als Zumischkomponente in dem Verfahren oder als Additiv nachträglich zu anderen Granulaten einzusetzen. Insbesondere die bevorzugten schwereren Granulate mit Schüttgewichten oberhalb 600 g/l enthalten vorzugsweise Komponenten, welche das Einspülverhalten und/oder das Löseverhalten der Granulate verbessern. Vorteilhafterweise werden hierzu alkoxylierte Fettalkohole mit 12 bis 80 Mol Ethylenoxid pro Mol Alkohol, beispielsweise Talgfettalkohol mit 14 EO, 30 EO oder 40 EO, und Polyethylenglykole mit einer relativen Molekülmasse zwischen 200 und 12000, vorzugsweise zwischen 200 und 600, eingesetzt.

Ebenso ist es möglich und kann in Abhängigkeit von der Rezeptur von Vorteil sein, wenn weitere einzelne Bestandteile des Mittels, beispielsweise Citrat bzw. Citronensäure oder andere Polycarboxylate bzw. Polycarbonsäuren, polymere Polycarboxylate, Zeolith und/oder Schichtsilikate, die gegebenenfalls kristallin sein können, nachträglich zu sprühgetrockneten, granulierten und/oder extrudierten Komponenten, die gegebenenfalls mit nichtionischen Tensiden und/oder anderen bei der Verarbeitungstemperatur flüssigen bis wachsartigen Inhaltsstoffen beaufschlagt sind, hinzugemischt werden. Bevorzugt ist dabei ein Verfahren, bei dem die Oberfläche von Teilkomponenten des Mittels oder des gesamtem Mittels zur Reduzierung der Klebrigkeit der an Niotensiden reichen Granulate und/oder zu ihrer verbesserten Löslichkeit nachträglich behandelt wird. Geeignete Oberflächenmodifizierer sind dabei aus dem Stand der Technik bekannt. Neben weiteren geeigneten sind dabei feinteilige Zeolithe, Kieselsäuren, amorphe Silikate, Fettsäuren oder Fettsäuresalze, beispielsweise Calciumstearat, insbesondere jedoch Mischungen aus Zeolith und Kieselsäuren oder Zeolith und Calciumstearat besonders bevorzugt.

### Kosmetische und/oder pharmazeutische Zubereitungen

Die erfindungsgemäßen verzweigten, weitgehend ungesättigten Fettalkoholpolyglycolether können auch zur Herstellung von kosmetischen und/oder pharmazeutischen Zubereitungen, wie beispielsweise Haarshampoos, Haarlotionen, Schaumbäder, Duschbäder, Cremes, Gele, Lotionen, alkoholische und wäßrig/alkoholische Lösungen, Emulsionen, Wachs/ Fett-Massen, Stiftpräparaten, Pudern oder Salben dienen. Diese Mittel können ferner als weitere Hilfs- und Zusatzstoffe milde Tenside, Ölkörper, Emulgatoren, Überfettungsmittel, Perlglanzwachse, Konsistenzgeber, Verdickungsmittel, Polymere, Siliconverbindungen, Fette, Wachse, Stabilisatoren, biogene Wirkstoffe, Deodorantien, Antitranspirantien, Antischuppenmittel, Filmbildner, Quellmittel, UV-Lichtschutzfaktoren, Antioxidantien, Hydrotrope, Konservierungsmittel, Insektenrepellentien, Selbstbräuner, Solubilisatoren, Parfümöle, Farbstoffe und dergleichen enthalten.

Typische Beispiele für geeignete milde, d.h. besonders hautverträgliche **Tenside** sind Fettalkoholsulfate, Fettalkoholethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine und/oder Proteinfettsäurekondensate, letztere vorzugsweise auf Basis von Weizenproteinen.

Als **Ölkörper** kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von Hydroxycarbonsäuren mit linearen oder verzweigten C₆-C₂₂-Feftalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetaikoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Feftalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, Guerbetcarbonate, Ester der Benzoesäure mit linearen und/oder verzweigten C₆-C₂₂-Alkoholen (z.B. Finsolv^{®} TN), lineare oder verzweigte, symmetrische oder unsymmetrische Dialkylether mit 6 bis 22 Kohlenstoffatomen pro Alkylgruppe, Ringöffnungsprodukte von epoxidierten Fettsäureestern mit Polyolen, Siliconöle und/oder aliphatische bzw. naphthenische Kohlenwasserstoffe, wie z.B. wie Squalan, Squalen oder Dialkylcyclohexane in Betracht.

Als **Emulgatoren** kommen beispielsweise nichtionogene Tenside aus mindestens einer der folgenden Gruppen in Frage:
➢ Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
➢ Alkyl- und/oder Alkenyloligoglykoside mit 8 bis 22 Kohlenstoffatomen im Alk(en)ylrest und deren ethoxylierte Analoga;
➢ Anlagerungsprodukte von 1 bis 15 Mol Ethylenoxid an Ricinusöl und/oder gehärtetes Ricinusöl;
➢ Anlagerungsprodukte von 15 bis 60 Mol Ethylenoxid an Ricinusöl und/oder gehärtetes Ricinusöl;
➢ Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
➢ Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
➢ Mischester aus Pentaerythrit, Fettsäuren, Citronensäure und Fettalkohol gemäß **DE 1165574 PS** und/oder Mischester von Fettsäuren mit 6 bis 22 Kohlenstoffatomen, Methylglucose und Polyolen, vorzugsweise Glycerin oder Polyglycerin.
➢ Mono-, Di- und Trialkylphosphate sowie Mono-, Di- und/oder Tri-PEG-alkylphosphate und deren Salze;
➢ Wollwachsalkohole;
➢ Polysiloxan-Polyalkyl-Polyether-Copolymere bzw. entsprechende Derivate;
➢ Polyalkylenglycole sowie
➢ Glycerincarbonat:

Die **Anlagerungsprodukte von Ethylenoxid und/oder von Propylenoxid** an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE 2024051 PS** als Rückfettungsmittel für kosmetische Zubereitungen bekannt.

**Alkyl- und/oder Alkenyloligoglycoside,** ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 Kohlenstoffatomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Typische Beispiele für geeignete **Partialglyceride** sind Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.

Als **Sorbitanester** kommen Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.

Typische Beispiele für geeignete **Polyglycerinester** sind Polyglyceryl-2 Dipolyhydroxystearate (Dehymuls^{®} PGPH), Polyglycerin-3-Diisostearate (Lameform^{®} TGI), Polyglyceryl-4 Isostearate (Isolan^{®} GI 34), Polyglyceryl-3 Oleate, Diisostearoyl Polyglyceryl-3 Diisostearate (Isolan^{®} PDI), Polyglyceryl-3 Methylglucose Distearate (Tego Care^{®} 450), Polyglyceryl-3 Beeswax (Cera Bellina^{®}), Polyglyceryl-4 Caprate (Polyglycerol Caprate T2010/90), Polyglyceryl-3 Cetyl Ether (Chimexane^{®} NL), Polyglyceryl-3 Distearate (Cremophor^{®} GS 32) und Polyglyceryl Polyricinoleate (Admul^{®} WOL 1403) Polyglyceryl Dimerate Isostearate sowie deren Gemische.

Beispiele für weitere geeignete **Polyolester** sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.

Weiterhin können als Emulgatoren **zwitterionische Tenside** verwendet werden..Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acyfaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin.

Schließlich kommen auch **Kationtenside** als Emulgatoren in Betracht, wobei solche vom Typ der Esterquats, vorzugsweise methylquaternierte Difettsäuretriethanolaminester-Salze, besonders bevorzugt sind.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als **Perlglanzwachse** kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

Als **Konsistenzgeber** kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten.

Geeignete **Verdickungsmittel** sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, (z.B. Carbopole^{®} von Goodrich oder Synthalene^{®} von Sigma), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete **kationische Polymere** sind beispielsweise kationische Cellulosederivate, wie z.B. eine quaternierte Hydroxyethylcellulose, die unter der Bezeichnung Polymer JR 400^{®} von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat^{®} (BASF), Kondensationsprodukte von Polyglycolen und Aminen, quaternierte Kollagenpolypeptide, wie beispielsweise Lauryldimonium hydroxypropyl hydrolyzed collagen (Lamequat^{®}L/Grünau), quaternierte Weizenpolypeptide, Polyethylenimin, kationische Siliconpolymere, wie z.B. Amidomethicone, Copolymere der Adipinsäure und Dimethylaminohydroxypropyldiethylentriamin (Cartaretine^{®}/Sandoz), Copolymere der Acrylsäure mit Dimethyldiallylammoniumchlorid (Merquat^{®} 550/Chemviron), Polyaminopolyamide, wie z.B. beschrieben in der FR 2252840 A sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar^{®} CBS, Jaguar^{®} C-17, Jaguar^{®} C-16 der Firma Celanese, quaternierte Ammoniumsalz-Polymere, wie z.B. Mirapol^{®} A-15, Mirapol^{®} AD-1, Mirapol^{®} AZ-1 der Firma Miranol.

Als **anionische, zwitterionische, amphotere und nichtionische Polymere** kommen beispielsweise Vinylacetat/Crotonsäure-Copolymere, VinylpyrrolidonNinylacrylat-Copolymere, Vinylacetat/Butylmaleat/lsobomylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/Acrylat-Copolymere, Octylacrylamid/methylmethacrylat/tert.Butylaminoethylmethacrylat/2-Hydroxyproylmethacrylat-Copolymere, Polyvinylpyrrolidon, Vinylpyrrolidon/Vinylacetat-Copolymere, Vinylpyrrolidon/Dimethylaminoethylmethacrylat/Vinylcaprolactam-Terpolymere sowie gegebenenfalls derivatisierte Celluloseether und Silicone in Frage.

Geeignete **Siliconverbindungen** sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt. Eine detaillierte Übersicht über geeignete flüchtige Silicone findet sich zudem von Todd et al. in **Cosm.Toil. 91, 27 (1976).**

Typische Beispiele für Fette sind Glyceride, als **Wachse** kommen u.a. natürliche Wachse, wie z.B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage.

Als **Stabilisatoren** können Metallsalze von Fettsäuren, wie z.B. Magnesium-, Aluminium- und/oder Zinkstearat bzw. -ricinoleat eingesetzt werden.

Unter **biogenen Wirkstoffen** sind beispielsweise Tocopherol, Tocopherolacetat, Tocopherolpalmitat, Ascorbinsäure, Desoxyribonucleinsäure, Retinol, Bisabolol, Allantoin, Phytantriol, Panthenol, AHA-Säuren, Aminosäuren, Ceramide, Pseudoceramide, essentielle Öle, Pflanzenextrakte und Vitaminkomplexe zu verstehen.

Kosmetische **Deodorantien** (Desodorantien) wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als keimhemmende Mittel, Enzyminhibitoren, Geruchsabsorber oder Geruchsüberdecker fungieren.

Als **keimhemmende Mittel** sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4 dichlorphenyl)hamstoff, 2,4,4'-Trichlor-2'-hydroxydiphenylether (Triclosan), 4-Chlor-3,5-dimethylphenol, 2,2'-Methylen-bis(6-brom-4-chlorphenol), 3-Methyl-4-(1-methylethyl)phenol, 2-Benzyl-4-chlorphenol, 3-(4-Chlorphenoxy)-1,2-propandiol, 3-lod-2-propinylbutylcarbamat, Chlorhexidin, 3,4,4'-Trichforcarbanilid (TTC), antibakterielle Riechstoffe, Thymol, Thymianöl, Eugenol, Nelkenöl, Menthol, Minzöl, Farnesol, Phenoxyethanol, Glycerinmonolaurat (GML), Diglycerinmonocaprinat (DMC), Salicylsäure-N-alkylamide wie z. B. Salicylsäure-n-octylamid oder Salicylsäure-n-decylamid.

Als **Enzyminhibitoren** sind beispielsweise Esteraseinhibitoren geeignet. Hierbei handelt es sich vorzugsweise um Trialkylcitrate wie Trimethylcitrat, Tripropylcitrat, Trüsopropylcitrat, Tributylcitrat und insbesondere Triethylcitrat (Hydagen^{®} CAT, Henkel KGaA, Düsseldorf/FRG). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbnonsäuren und deren Ester wie beispielsweise Citronensäure, Äpfelsäure, Weinsäure oder Weinsäurediethylester, sowie Zinkglycinat.

Als **Geruchsabsorber** eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, daß dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verfeihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, p-tert.-Butylcyclohexylacetat, Linalylacetat, Phenylethylacetat, Linalylbenzoat, Benzylformiat, Allylcyclohexylpropionät, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, lraldein gamma, Phenylessigsäure, Geranylacetat, Benzylacetat, Rosenoxid, Romilat, Irotyl und Floramat allein oder in Mischungen, eingesetzt.

**Antitranspirantien** (Antiperspirantien) reduzieren durch Beeinflussung der Aktivität der ekkrinen Schweißdrüsen die Schweißbildung, und wirken somit Achselnässe und Körpergeruch entgegen. Wässrige oder wasserfreie Formulierungen von Antitranspirantien enthalten typischerweise folgende Inhaltsstoffe:
➢ adstringierende Wirkstoffe,
➢ Ölkomponenten,
➢ nichtionische Emulgatoren,
➢ Coemulgatoren,
➢ Konsistenzgeber,
➢ Hilfsstoffe wie z. B. Verdicker oder Komplexierungsmittel und/oder
➢ nichtwässrige Lösungsmittel wie z. B. Ethanol, Propylenglykol und/oder Glycerin.

Als adstringierende Antitranspirant-Wirkstoffe eignen sich vor allem Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium-Zirkonium-Trichlorohydrat, Aluminium-Zirkonium-tetrachlorohydrat, Aluminium-Zirkonium-pentachlorohydrat und deren Komplexverbindungen z. B. mit Aminosäuren wie Glycin. Daneben können in Antitranspirantien übliche öllösliche und wasserlösliche Hilfsmittel in geringeren Mengen enthalten sein. Solche öllöslichen Hilfsmittel können z.B. sein:
➢ entzündungshemmende, hautschützende oder wohlriechende ätherische Öle,
➢ synthetische hautschützende Wirkstoffe und/oder
➢ öllösliche Parfümöle.

Übliche wasserlösliche Zusätze sind z.B. Konservierungsmittel, wasserlösliche Duftstoffe, pH-Wert-Stellmittel, z.B. Puffergemische, wasserlösliche Verdickungsmittel, z.B. wasserlösliche natürliche oder synthetische Polymere wie z.B. Xanthan-Gum, Hydroxyethylcellulose, Polyvinylpyrrolidon oder hochmolekulare Polyethylenoxide.

Als **Antischuppenmittel** können Climbazol, Octopirox und Zinkpyrethion eingesetzt werden.

Gebräuchliche **Filmbildner** sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

Als **Quellmittel** für wäßrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R.Lochhead in Cosm.Toil. 108, 95 (1993) entnommen werden.

Unter **UV-Lichtschutzfaktoren** sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
➢ 3-Benzylidencampher bzw. 3-Benzylidennorcampher und dessen Derivate, z.B. 3-(4-Methylbenzyliden)campher wie in der **EP 0693471 B1** beschrieben;
➢ 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
➢ Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
➢ Ester der Salicylsäure, vorzugsweise Salicylsäure -2-ethylhexylester, Salicylsäure-4-isopropylbenzylester, Salicylsäurehomomenthylester;
➢ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
➢ Ester der Benzalmalonsäure, vorzugsweise 4-Methoxybenzmalonsäuredi-2-ethylhexylester;
➢ Triazinderivate, wie z.B. 2,4,6-Trianilino-(p-carbo-2'-ethyl-1'-hexyloxy)-1,3,5-triazin und Octyl Triazon, wie in der **EP 0818450 A1** beschrieben oder Dioctyl Butamido Triazone (Uvasorb^{®} HEB);
➢ Propan-1,3-dione, wie z.B. 1-(4-tert.Butylphenyl)-3-(4'methoxyphenyl)propan-1,3-dion;
➢ Ketotricyclo(5.2.1.0)decan-Derivate, wie in der **EP 0694521 B1** beschrieben.

Als wasserlösliche Substanzen kommen in Frage:
➢ 2-Phenylbenzimidazol-5-sulfonsäure und deren Alkali-, Erdalkali-, Ammonium-, Alkylammonium-, Alkanolammonium- und Glucammoniumsalze;
➢ Sulfonsäurederivate von Benzophenonen, vorzugsweise 2-Hydroxy-4-methoxybenzophenon-5-sulfonsäure und ihre Salze;
➢ Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol 1789), 1-Pheny4-3-(4'-isopropylphenyl)-propan-1,3-dion sowie Enaminverbindungen, wie beschrieben in der **DE 19712033 A1** (BASF). Die UV-A und UV-B-Filter können selbstverständlich auch in Mischungen eingesetzt werden. Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex^{®} T2000 (Merck). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet. Weitere geeignete UV-Lichtschutzfilter sind der Übersicht von P.Finkel in **SÖFW-Journal 122, 543 (1996)** zu entnehmen.

Neben den beiden vorgenannten Gruppen primärer Lichtschutzstoffe können auch sekundäre Lichtschutzmittel vom Typ der **Antioxidantien** eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.
Zur Verbesserung des Fließverhaltens können ferner **Hydrotrope**, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
➢ Glycerin;
➢ Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
➢ technische Oligoglyceringemische mit einem Eigenkondensationsgrad von 1,5 bis 10 wie etwa technische Diglyceringemische mit einem Diglyceringehalt von 40 bis 50 Gew.-%;
➢ Methyolverbindungen, wie insbesondere Trimethylolethan, Trimethylolpropan, Trimethylolbutan, Pentaerythrit und Dipentaerythrit;
➢ Niedrigalkylglucoside, insbesondere solche mit 1 bis 8 Kohlenstoffen im Alkylrest, wie beispielsweise Methyl- und Butylglucosid;
➢ Zuckeralkohole mit 5 bis 12 Kohlenstoffatomen, wie beispielsweise Sorbit oder Mannit,
➢ Zucker mit 5 bis 12 Kohlenstoffatomen, wie beispielsweise Glucose oder Saccharose;
➢ Aminozucker, wie beispielsweise Glucamin;
➢ Dialkoholamine, wie Diethanolamin oder 2-Amino-1,3-propandiol.

Als **Konservierungsmittel** eignen sich beispielsweise Phenoxyethanol, Formaldehydlösung, Parabene, Pentandiol oder Sorbinsäure sowie die in Anlage 6, Teil A und B der Kosmetikverordnung aufgeführten weiteren Stoffklassen. Als **insekten-Repellentien** kommen N,N-Diethyl-m-toluamid, 1,2-Pentandiol oder Ethyl Butylacetylaminopropionate in Frage, als **Selbstbräuner** eignet sich Dihydroxyaceton.

Als **Parfümöle** seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, ∝-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexyizimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, lso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt - oder Heißprozesse erfolgen; vorzugsweise arbeitet man nach der Phaseninversionstemperatur-Methode.

In einer weiteren Ausführungsform werden die weitgehend ungesättigten Fettalkoholpolyglycolether in Emulsionen für Schmierstoffanwendungen eingesetzt. Außerdem finden die erfindungsgemäßen Fettalkoholpolyglycolether Verwendung in Mitteln zur Bekämpfung von Moskitolarven.

### Beispiele

**Beispiel 1.** 23 kg Monomerfettsäure Edenor^{®} 935 (Henkel KGaA) wurden mit 20 kg Methanol 2 h bei 240 °C und 100 bar verestert Nach Abtrennen der Wasser/Methanol-Mischung wurde die gleiche Menge Frisch-Methanol zugesetzt und der Vorgang zweimal wiederholt. Der so erhaltene Ester besaß eine Säurezahl von 0,8. Der Methylester wurde in der Festbettfahrweise an einem Zn-Cr-Katalysator unter Erhalt der Doppelbindung hydriert. Hierbei wurden pro Stunde 0,5 Volumeneinheiten Methylester - bezogen auf das Gesamtvolumen der Anlage - durchgesetzt. Nach Abtrieb des Methanols wurde der Rohalkohol destilliert (3% Vorlauf, 90 % Hauptlauf, 6% Rückstand). Der resultierende Alkohol zeigte eine Hydroxylzahl von 192, eine Verseifungszahl von 0,9 und eine Iodzahl von 74; der Festpunkt betrug 25,8 °C. 293 g (1 Mol) des so erhaltenen Isooleylalkohols wurden in einem Rührautoklaven gegeben und bei 100 °C für ca. 45 Minuten getrocknet. Nach der Zugabe von 2 g Natriummethylat in Form einer 30 Gew.-%igen methanolischen Lösung als basischer Katalysator wurde die Ethoxylierung mit 440 g (10 Mol) Ethylenoxid bei 120 bis 160 °C und einem autogenen Druck von 5 bar durchgeführt. Nach Abkühlen der Reaktionsmischung wurde evakuiert, um Spuren nicht umgesetzten Ethylenoxids zu beseitigen. Das Isooleylalkohol+10EO-Addukt besaß eine lodzahl von 28.

**Beispiel 2.** Monomerfettsäure wurde durch Kristallisation aus Methanol/Wasser (Emersol-Verfahren) von geradkettigen, gesättigten Fettsäuren weitgehend befreit. Auf diese Weise wurden ca. 20 Gew.% Fettsäure, überwiegend Palmitin- und Stearinsäure, abgetrennt. Die nach Abdestillation des Lösemittels erhaltene flüssige Fettsäuremischung besaß einen Titer von 5 °C und wurde analog Beispiel 1 zunächst in den Methylester überführt und dann zum ungesättigten Fettalkohol hydriert. Dieser zeigte eine Hydroxylzahl von 191, eine Verseifungszahl von 1,7 und eine lodzahl von 87; der Festpunkt betrug 3,8 °C. 293 g (1 Mol) des so erhaltenen Isooleylalkohols wurden in einem Rührautoklaven gegeben und bei 100 °C für ca. 45 Minuten getrocknet. Nach der Zugabe von 2 g Natriummethylat in Form einer 30 Gew.-%igen methanolischen Lösung als basischer Katalysator wurde die Ethoxylierung mit 308 g (7 Mol) Ethylenoxid bei 120 bis 160 °C und einem autogenen Druck von 5 bar durchgeführt. Nach Abkühlen der Reaktionsmischung wurde evakuiert, um Spuren nicht umgesetzten Ethylenoxids zu beseitigen. Das Isooleylalkohol+7EO-Addukt besaß eine lodzahl von 41.

In den nachfolgenden Tabellen 1 und 2 sind eine Reihe von Formulierungsbeispiele angegeben.

**Tabelle 1**

| Detergenszubereitungen (Wasser, Konservierungsmittel ad 100 Gew.-%) | | | | | | | | | | |
|---|---|---|---|---|---|---|---|---|---|---|
| **Zusammensetzung** | **1** | **2** | **3** | **4** | **5** | **6** | **7** | **8** | **9** | **10** |
| **Maranil**^{**®**} **Paste A 55** | - | 23,6 | - | 19,0 | - | - | 11,0 | 7,0 | - | - |
| Dodecylhenzolsulfonat, Natriumsalz | | | | | | | | | | |
| **Sulfopon**^{**®**} **1218 W** | 5,5 | - | 14,5 | - | - | 10,0 | - | 3,0 | 12 | 350 |
| Laurylstearylsulfat, Natriumsalz | | | | | | | | | | |
| **Dehyquart**^{**®**} **AU 56** | - | - | - | - | 6,7 | - | - | - | - | - |
| Methylquatemierter Diplamiettsäuretriethanolammoniumester, Methosulfat | | | | | | | | | | |
| **Comperlan**^{**®**} **100** | - | - | - | - | - | - | - | - | - | 10,0 |
| Kokosfettsäureethanolamid | | | | | | | | | | |
| **Fettalkoholpolyglycolether gemäß Bsp.2** | 5,0 | 1,0 | 1,0 | 1,0 | 5,0 | 5,0 | 8,0 | 1,0 | 3,0 | 1,0 |
| **Dehydol**^{**®**} **08** | - | - | - | - | | 25,0 | - | - | - | - |
| Octanol+8EO | | | | | | | | | | |
| **Dehydol**^{**®**} **LT5** | - | - | - | - | - | - | 2,0 | - | 4,0 | - |
| Laurylstearylalkohol+5EO | | | | | | | | | | |
| **Dehydol**^{**®**} **LT7** | 7,0 | - | - | - | 5,0 | - | - | 2,0 | 4,0 | - |
| Laurylstearylalkohol+7E0 | | | | | | | | | | |
| **Eumulgin**^{**®**} **WO 7** | - | - | 10,0 | 4,0 | - | - | - | - | - | - |
| Oleylalcohol+7EO | | | | | | | | | | |
| **Eumulgin**^{**®**} **RT 40** | - | - | - | - | - | - | - | - | - | 5,0 |
| Castoröl+40EO | | | | | | | | | | |
| **Glucopon**^{**®**} **600 CS UP** | 4,0 | 5,0 | 11,0 | 15,0 | 6,0 | 19,5 | - | - | 1,0 | - |
| Laurylglucosid | | | | | | | | | | |
| **Edenor**^{**®**} **K 1218** | 6,0 | - | 4,0 | - | - | 12,0 | - | - | - | - |
| Kokosfettsäure | | | | | | | | | | |
| **Sokalan**^{**®**} **CP5** | - | - | - | 5,0 | - | - | - | 4,0 | 5,5 | - |
| Maleinsäure-Acrylsäure-Copolymer, Natriumsalz | | | | | | | | | | |
| **Natriumcarbonat** | - | 2,0 | - | 7,0 | - | - | 13,0 | 7,0 | 10,0 | - |
| **Natriumsulfat** | - | - | - | 19,0 | - | - | - | 10,0 | 9,5 | 45,0 |
| **Natriumtripolyphosphat** | - | 20,0 | - | - | - | - | - | - | - | - |
| **Zeolith A** | - | - | - | 25,0 | - | - | 39,0 | 25,0 | 32,5 | - |
| **Amylase** | - | - | - | - | - | - | 0,2 | - | 0,2 | - |
| **Cellulase** | - | - | - | - | - | - | 0,2 | - | - | - |
| **Lipase** | 0,8 | - | - | - | - | - | 0,2 | 0,7 | 0,1 | - |
| **Protease** | - | - | - | - | - | - | 0,2 | 0,3 | 0,1 | - |
| **Glycerin** | 3,0 | - | - | - | - | - | - | - | - | - |
| **Propylenglycol** | 7,0 | - | 5,0 | - | - | - | - | - | - | - |
| **Ethanol** | 5,0 | - | 5,0 | - | - | 7,0 | - | - | - | - |
| **Protil**^{**®**} **N** | - | - | - | 3,0 | - | - | - | - | - | - |
| Natriumsilicat | | | | | | | | | | |
| **Dehydran**^{**®**} **760** | - | - | - | 7,0 | - | - | 4,0 | 5,0 | 4,5 | - |
| Silicon/Paraffin on carrier | | | | | | | | | | |
| **Polyvinylpyrrolidon** | - | - | - | - | - | - | 0,3 | 1,0 | 0,8 | - |
| **Natriumhydroxid** | 5,5 | - | 5,0 | - | - | 7,6 | - | - | - | - |
| **Natriumhydrogencarbonat** | - | - | - | - | - | - | 7,0 | 9,0 | 5,5 | - |
| **Natriumcitrat** | 0,1 | - | - | - | 0,1 | - | 4,0 | 12,0 | 4,0 | 5,0 |

| **Zusammensetzung** | **11** | **12** | **13** | **14** | **15** | **16** | **17** | **18** | **19** | **20** |
|---|---|---|---|---|---|---|---|---|---|---|
| **Maranil**^{**®**} **Paste A 55** | 20,0 | - | - | - | - | - | - | - | - | 6,0 |
| Dodecylbenzolsulfonat, Natriumsalz | | | | | | | | | | |
| **Texapon**^{**®**} **N 70** | 3,0 | 15,0 | 10,0 | - | 20,0 | - | - | - | - | - |
| Laurylalkohol+2EO-sulfat, Natriumsalz | | | | | | | | | | |
| **Texapon**^{**®**} **LS 35** | - | 10,0 | 2,0 | 5,0 | - | - | - | - | 2,0 | |
| Laurylalkohol+3.5EO-sulfat, Natriumsalz | | | | | | | | | | |
| **Texapon**^{**®**} **842** | 5,0 | 3,5 | - | - | | - | - | - | - | - |
| Octylsulfat, Natriumsalz | | | | | | | | | | |
| **Alkansulfonat C 13/17** | - | - | - | 12,5 | 18,0 | - | - | - | - | - |
| Glucopon^{®} 600 CS UP | 3,0 | 2,0 | 15,0 | 4,0 | - | - | - | 1,0 | - | - |
| Laurylglucosid | | | | | | | | | | |
| **Fettalkoholpolyglycolether gemäß Bsp.2** | 1,0 | 1,0 | 1,0 | 1,0 | 1,0 | 1,0 | 1,0 | 1,0 | 1,0 | 1,0 |
| **Dehydol**^{**®**} **O10** | - | - | - | - | 8,0 | - | - | - | - | - |
| Octanol+10EO | | | | | | | | | | |
| **Dehydol**^{**®**} **980** | - | - | - | - | - | - | - | - | 1,0 | 2,0 |
| Laurylmyristylalkohol+1PO+6E0 | | | | | | | | | | |
| **Dehypon**^{**®**} **LS 54** | - | - | - | - | - | 15,0 | - | - | - | - |
| Laurylalkohol+5EO+4PO | | | | | | | | | | |
| **Dehypon**^{**®**} **LS** 104 | - | - | - | - | - | - | 10,0 | - | - | - |
| Laurylalkohol+10EO-butylether | | | | | | | | | | |
| **Dehyton**^{**®**} **K** | - | 3,5 | 5,0 | - | - | - | - | - | - | - |
| Kokosfettsäureamidopropylbetain | | | | | | | | | | |
| **Sokalan**^{**®**} **DCS** | - | - | 2,5 | - | - | - | - | - | - | - |
| Bemsteinsäure-Glutarsäure-Adipinsäure-Gemisch | | | | | | | | | | |
| **Ethanol** | - | - | 8,0 | - | - | - | - | - | - | - |
| **Isopropylalkohol** | - | - | - | - | - | - | - | - | - | 3,0 |
| **Butyldiglycol** | - | - | - | - | - | - | - | - | - | 1,5 |
| **Cumolsulfonat, Natriumsalz** | - | - | - | - | - | 20,0 | 13,0 | - | - | - |
| **Natriumhydroxid** | - | - | 1,5 | - | - | - | - | - | - | |
| **Ammoniak** | - | - | - | - | - | - | - | - | - | 0,5 |
| **Wasserstoffperoxid** | - | - | - | - | - | - | - | 5,0 | 5,0 | - |
| **Turpinal**^{**®**} **4 NL** | - | - | - | - | - | - | - | 0,3 | 0,3 | - |
| Tetranatriumeditronal | | | | | | | | | | |
| **Citronensäure** | 0,2 | 0,2 | 0,2 | 0,2 | 0,2 | - | 5,0 | - | - | - |

| | | | | | | | | | | |
|---|---|---|---|---|---|---|---|---|---|---|
| (1-6) Light Duty Detergent (7-9) Heavy Duty detergent (10) Toilettenstein (11-15) Handgeschirrspülmittel (16, 17) Maschinengeschirrspülmittel (18-20) Reinigungsmittel | | | | | | | | | | |

**Tabelle 2**

| Kosmetische Zubereitungen (Wasser, Konservierungsmittel ad 100 Gew.-%) | | | | | | | | | | |
|---|---|---|---|---|---|---|---|---|---|---|
| **Zusammensetzung (INCI)** | **21** | **22** | **23** | **24** | **25** | **26** | **27** | **28** | **29** | **30** |
| **Texapon**^{**®**} **NSO** | - | - | - | - | - | - | 38,0 | 38,0 | 25,0 | - |
| Sodium Laureth Sulfate | | | | | | | | | | |
| **Texapon**^{**®**} **SB** 3 | - | - | - | - | - | - | - | - | 10,0 | - |
| Disodium Laureth Sulfosuccinate | | | | | | | | | | |
| **Plantacare**^{**®**} **818** | - | - | - | - | - | - | 7,0 | 7,0 | 6,0 | - |
| Coco Glucosides | | | | | | | | | | |
| **Plantacare**^{**®**} **PS 10** | - | - | - | - | - | - | - | - | - | 12,0 |
| Sodium Laureth Sulfate (and) Coco Glucosides | | | | | | | | | | |
| **Dehyton**^{**®**} **PK 45** | - | - | - | - | - | - | - | - | 10,0 | - |
| Cocamidopropyl Betaine | | | | | | | | | | |
| **Dehyquart**^{**®**} **A** | 2,0 | 2,0 | 2,0 | 2,0 | 4,0 | 4,0 | - | - | - | - |
| Cetrimonium Chloride | | | | | | | | | | |
| **Dehyquart L**^{**®**} **80** | 1,2 | 1,2 | 1,2 | 1,2 | 0,6 | 0,6 | - | - | - | - |
| Dicocoylmethylethoxymonium Methosulfate (and) Propylenglycol | | | | | | | | | | |
| **Fettalkoholpolyglycolether gemäß Bsp.1** | 1,0 | 1,0 | 1,0 | 1,0 | 1,0 | 1,0 | 1,0 | 1,0 | 1,0 | 1,0 |
| **Eumulgin**^{**®**} **B2** | 0,8 | 0,8 | - | 0,8 | - | 1,0 | - | - | - | - |
| Ceteareth-20 | | | | | | | | | | |
| **Eumulgin**^{**®**} **VL 75** | - | - | 0,8 | - | 0.8 | - | - | - | - | - |
| Lauryl Glucoside (and) Polyglyceryl-2 Polyhydroxystearate (and) Glycerin | | | | | | | | | | |
| **Lanette**^{**®**}**0** | 2,5 | 2,5 | 2,5 | 2,5 | 3,0 | 2,5 | - | - | - | - |
| Cetearyl Alcohol | | | | | | | | | | |
| **Cutina**^{**®**} **GMS** | 0,5 | 0,5 | 0,5 | 0,5 | 0,5 | 1,0 | - | - | - | - |
| Glyceryl Stearate | | | | | - | | | | | |
| **Cetiol**^{**®**} **HE** | 1,0 | - | - | - | - | - | - | - | 1,0 | |
| PEG-7 Glyceryl Cocoate | | | | | | | | | | |
| **Cetiol**^{**®**} **PGL** | - | 1,0 | - | - | 1,0 | - | - | - | - | - |
| Hexyldecanol (and) Hexyldecyl Laurate | | | | | | | | | | |
| **Eutanol**^{**®**} **G** | - | - | 1,0 | 1,0 | - | 1,0 | - | - | - | - |
| Octyldodecanol | | | | | | | | | | |
| **Nutrilan**^{**®**} **Keratin W** | - | - | - | 2,0 | - | - | - | - | - | - |
| Hydrolyzed Keratin | | | | | | | | | | |
| **Lamesoft**^{**®**} **LMG** | - | - | - | - | - | - | 3,0 | 2,0 | 4,0 | - |
| Glyceryl Laurate (and) Potassium Cocoyl Hydrolyzed Collagen | | | | | | | | | | |
| **Euperlan**^{**®**} **PK 3000 AM** | - | - | - | - | - | - | - | 3,0 | 5,0 | 5,0 |
| Glycol Distearate (and) Laureth-4 (and) Cocamidopropyl Betaine | | | | | | | | | | |
| **Generol**^{**®**} **122 N** | - | - | - | - | 1,0 | 1,0 | - | - | - | - |
| Soja Sterol | | | | | | | | | | |
| **Highcareen**^{**®**} **GS** | 1,0 | 1,0 | 1,0 | 1,0 | 1,0 | 1,0 | 1,0 | 1,0 | 1,0 | 1,0 |
| Betaglucan | | | | | | | | | | |
| **Hydagen**^{**®**} **CMF** | 1,0 | 1,0 | 1,0 | 1,0 | 1,0 | 1,0 | 1,0 | 1,0 | 1,0 | 1,0 |
| Chitosan | | | | | | | | | | |
| **Copherol**^{**®**} **12250** | - | - | 0,1 | 0,1 | - | - | - | - | - | - |
| Tocopherol Acetate | | | | | | | | | | |
| **Arlypon**^{**®**} F | - | - | - | - | - | - | 3,0 | 3,0 | 1,0 | - |
| Laureth-2 | | | | | | | | | | |
| **Sodium Chloride** | - | - | - | - | - | - | - | 1,5 | - | 1,5 |

| **Zusammensetzung (INCI)** | **31** | **32** | **33** | **34** | **35** | **36** | **37** | **38** | **39** | **40** |
|---|---|---|---|---|---|---|---|---|---|---|
| **Texapon**^{**®**} **NSO** | 15,0 | 15,0 | 10,0 | - | 20,0 | 8,0 | - | - | - | - |
| Sodium Laureth Sulfate | | | | | | | | | | |
| **Texapon**^{**®**} **K 14 S** | - | - | - | - | - | - | - | - | 8,0 | 15,0 |
| Sodium Myreth Sulfate | | | | | | | | | | |
| **Texapon**^{**®**} **SB 3** | - | - | - | - | - | 7,0 | - | - | - | - |
| Disodium Laureth Sulfosuccinate | | | | | | | | | | |
| **Plantacare**^{**®**} **818** | 5,0 | 5,0 | 4,0 | - | - | - | - | - | 6,0 | 4,0 |
| Coco Glucosides | | | | | | | | | | |
| **Plantacare**^{**®**} **2000** | - | - | - | - | 5,0 | 4,0 | - | - | - | - |
| Decyl Glucoside | | | | | | | | | | |
| **Plantacare**^{**®**} **PS 10** | - | - | - | 40,0 | - | - | 16,0 | 17,0 | - | - |
| Sodium Laureth Sulfate (and) Coco Glucosides | | | | | | | | | | |
| **Dehyton**^{**®**} **PK 45** | 20,0 | 20,0 | - | - | 8,0 | - | - | - | - | 7,0 |
| Cocamidopropyl Betaine | | | | | | | | | | |
| **Fettalkoholpolyglycolether gemäß Bsp.1** | 1,0 | 1,0 | 1,0 | 1,0 | 1,0 | 1,0 | 1,0 | 1,0 | 1,0 | 1,0 |
| **Eumulgin**^{**®**} **B1** | - | - | - | - | 1,0 | - | - | - | - | - |
| Ceteareth-12 | | | | | | | | | | |
| **Eumulgin**^{**®**} **B2** | - | - | - | 1,0 | - | - | - | - | - | - |
| Ceteareth-20 | | | | | | | | | | |
| **Lameform**^{**®**} **TGI** | - | - | - | 4,0 | - | - | - | - | - | - |
| Polyglyceryl-3 Isostearate | | | | | | | | | | |
| **Dehymuls**^{**®**} **PGPH** | - | - | 1,0 | - | - | - | - | - | - | - |
| Polyglyceryl-2 Dipolyhydroxystearate | | | | | | | | | | |
| **Monomuls**^{**®**} **90-L 12** | - | - | - | - | - | - | - | - | 1,0 | 1,0 |
| Glyceryl Laurate | | | | | | | | | | |
| **Cetiol**^{**®**} **HE** | - | 0,2 | - | - | - | - | - | - | - | - |
| PEG-7 Glyceryl Cocoate | | | | | | | | | | |
| **Eutanol**^{**®**} **G** | - | - | - | 1,0 | - | - | - | - | - | - |
| Octyldodecanol | | | | | | | | | | |
| **Nutrilan**^{**®**} **Keratin W** | - | - | - | - | - | - | - | - | 2,0 | 2,0 |
| Hydrolyzed Keratin | | | | | | | | | | |
| **Nutrilan**^{**®**} **I** | 1,0 | - | - | - | - | 2,0 | - | 2,0 | - | - |
| Hydrolyzed Collagen | | | | | | | | | | |
| **Lamesoft**^{**®**} **LMG** | - | - | - | - | - | - | | - | 1,0 | 5,0 |
| Glyceryl Laurate (and) Potassium Cocoyl Hydrolyzed Collagen | | | | | | | | | | |
| **Gluadin**^{**®**} **WK** | 1,0 | 1,5 | 4,0 | 1,0 | 3,0 | 1,0 | 2,0 | 2,0 | 2,0 | - |
| Sodium Cocoyl Hydrolyzed Wheat Protein | | | | | | | | | | |
| **Euperlan**^{**®**} **PK 3000 AM** | 5,0 | 3,0 | 4,0 | - | - | - | - | 3,0 | 3,0 | - |
| Glycol Distearate (and) Laureth-4 (and) Cocamidopropyl Betaine | | | | | | | | | | |
| **Panthenol** | - | - | 1,0 | - | - | - | - | - | - | - |
| **Arlypon**^{**®**} **F** | 2,6 | 1,6 | - | 1,0 | 1,5 | - | - | - | - | - |
| Laureth-2 | | | | | | | | | | |
| **Highcareen**^{**®**} **GS** | 1,0 | 1,0 | 1,0 | 1,0 | 1,0 | 1,0 | 1,0 | 1,0 | 1,0 | 1,0 |
| Betaglucan | | | | | | | | | | |
| **Hydagen**^{**®**} **CMF** | 1,0 | 1,0 | 1,0 | 1,0 | 1,0 | 1,0 | 1,0 | 1,0 | 1,0 | 1,0 |
| Chitosan | | | | | | | | | | |
| **Sodium Chloride** | - | - | - | - | - | 1,6 | 2,0 | 2,2 | - | 3,0 |
| Glycerin (86 Gew.-%ig) | - | 5,0 | - | - | - | - | - | 1,0 | 3,0 | - |

| **Zusammensetzung (INCI)** | **41** | **42** | **43** | **44** | **45** | **46** | **47** | **48** | **49** | **50** |
|---|---|---|---|---|---|---|---|---|---|---|
| Texapon^{®} NSO | - | 30,0 | 30,0 | - | 25,0 | - | - | - | - | - |
| Sodium Laureth Sulfate | | | | | | | | | | |
| Plantacare^{®} 818 | - | 10,0 | - | - | 20,0 | - | - | - | - | - |
| Coco Glucosides | | | | | | | | | | |
| Plantacare^{®} PS 10 | 22,0 | - | 5,0 | 22,0 | - | - | - | - | - | - |
| Sodium Laureth Sulfate (and) Coco Glucosides | | | | | | | | | | |
| Fettalkoholpolyglycolether gemäß Bsp.1 | 1,0 | 1,0 | 1,0 | 1,0 | 1,0 | 1,0 | 1,0 | 1,0 | 1,0 | 1,0 |
| Dehyton^{®} PK 45 | 15,0 | 10,0 | 15,0 | 15,0 | 20,0 | - | - | - | - | - |
| Cocamidopropyl Betaine | | | | | | | | | | |
| Emulgade^{®} SE | - | - | - | - | - | 5,0 | 5,0 | 4,0 | - | - |
| Glyceryl Sterate (and) Ceteareth 12/20 (and) Cetearyl Alcohol (and) Cetyl Palmitate | | | | | | | | | | |
| Lameform^{®} TGI | - | - | - | - | - | - | - | - | 4,0 | - |
| Polyglyceryl-3 Isostearate | | | | | | | | | | |
| Dehymuls^{®} PGPH | - | - | - | - | - | - | - | - | - | 4,0 |
| Polyglyceryl-2 Dipolyhydroxystearate | | | | | | | | | | |
| Monomuls^{®} 90-O 18 | - | - | - | - | - | - | - | - | 2,0 | - |
| Glyceryl Oleate | | | | | | | | | | |
| Cetiol^{®} HE | 2,0 | - | - | 2,0 | 5,0 | - | - | - | - | 2,0 |
| PEG-7 Glyceryl Cocoate | | | | | | | | | | |
| Cetiol^{®} OE | - | - | - | - | - | - | - | - | 5,0 | 6,0 |
| Dicaprylyl Ether | | | | | | | | | | |
| Cetiol^{®} PGL | - | - | - | - | - | - | - | 3,0 | 10,0 | 9,0 |
| Hexyldecanol (and) Hexyldecyl Laurate | | | | | | | | | | |
| Cetiol^{®} SN | - | - | - | - | - | 3,0 | 3,0 | - | - | - |
| Cetearyl Isononanoate | | | | | | | | | | |
| Cetiol^{®} V | - | - | - | - | - | 3,0 | 3,0 | - | - | - |
| Decyl Oleate | | | | | | | | | | |
| Myritol^{®} 318 | - | - | - | - | - | - | - | 3,0 | 5,0 | 5,0 |
| Coco Caprylate Caprate | | | | | | | | | | |
| Bees Wax | - | - | - | - | - | - | - | - | 7,0 | 5,0 |
| Nutrilan^{®} Elastin E20 | - | - | - | - | - | 2,0 | - | - | - | - |
| Hydrolyzed Elastin | | | | | | | | | | |
| Nutrilan^{®} I-50 | - | - | - | - | 2,0 | - | 2,0 | - | - | - |
| Hydrolyzed Collagen | | | | | | | | | | |
| Gluadin^{®} AGP | 0,5 | 0,5 | 0,5 | - | - | - | - | 0,5 | - | - |
| Hydrolyzed Wheat Gluten | | | | | | | | | | |
| Gluadin^{®} WK | 2,0 | 2,0 | 2,0 | 2,0 | 5,0 | - | - | - | 0,5 | 0,5 |
| Sodium Cocoyl Hydrolyzed Wheat Protein | | | | | | | | | | |
| Euperlan^{®} PK 3000 AM | 5,0 | - | - | 5,0 | - | - | - | - | - | - |
| Glycol Distearate (and) Laureth-4 (and) Cocamidopropyl Betaine | | | | | | | | | | |
| Highcareen^{®} GS | 1,0 | 1,0 | 1,0 | 1,0 | 1,0 | 1,0 | 1,0 | 1,0 | 1,0 | 1,0 |
| Betaglucan | | | | | | | | | | |
| Hydagen^{®} CMF | 1,0 | 1,0 | 1,0 | 1,0 | 1,0 | 1,0 | 1,0 | 1,0 | 1,0 | 1,0 |
| Chitosan | | | | | | | | | | |
| Magnesium Sulfate Hepta Hydrate | - | - | - | - | - | - | - | - | 1,0 | 1,0 |
| Glycerin (86 Gew.-%ig) | - | - | - | - | - | 3,0 | 3,0 | 5,0 | 5,0 | 3,0 |

| | | | | | | | | | | |
|---|---|---|---|---|---|---|---|---|---|---|
| (21-24) Haarspülung, (25-26) Haarkur, (27-28) Duschbad, (29) Duschgel, (30) Waschlotion (31-34) Duschbad "Two-in-One), (35-40) Shampoo (41-45) Schaumbad, (46) Softcreme, (47, 48) Feuchtigkeitsemulsion, (49, 50) Nachtcreme | | | | | | | | | | |

## Patentansprüche

1. Verfahren zur Herstellung von verzweigten, weitgehend ungesättigten Fettalkoholpolyglycolether, bei dem man
(a) ungesättigte Fettsäuren mit 16 bis 22 Kohlenstoffatomen in an sich bekannter Weise dimerisiert,
(b) die bei der Dimerisierung anfallende Monomerfraktion abtrennt,
(c) die in dieser Fraktion enthaltenen verzweigten, weitgehend ungesättigten Fettsäuren in die entsprechenden Fettsäuremethylester überführt,
(d) die verzweigten, weitgehend ungesättigten Fettsäuremethylester unter Erhalt der Doppelbindungen zu den entsprechenden verzweigten, weitgehend ungesättigten Fettalkoholen hydriert und diese
(e) in an sich bekannter Weise unter Erhalt der Doppelbindung alkoxyliert.

2. Verfahren nach Anspruch 1, **dadurch gekennzeichnet, dass** man die bei der Dimerisierung anfallende Monomerfraktion zunächst einer fraktionierten Kristallisation unterwirft und die dabei anfallende flüssige Phase gegebenenfalls nach Destillation der Veresterung unterwirft.

3. Verfahren nach den Ansprüchen 1 und/oder 2, **dadurch gekennzeichnet, dass** man die Methylester und/oder die Fettalkohole einer Destillation und/oder fraktionierten Kristallisation unterwirft.

4. Verfahren nach mindestens einem der Ansprüche 1 bis 3, **dadurch gekennzeichnet, daß** man an die verzweigten, weitgehend ungesättigten Fettalkohole durchschnittlich 1 bis 50 Mol Alkylenoxid anlagert.

5. Verwendung der verzweigten, weitgehend ungesättigten Fettalkoholpolyglycolether nach Anspruch 1 zur Herstellung von Wasch-, Spül-, Reinigungs- und Avivagemitteln.

6. Verwendung der verzweigten, weitgehend ungesättigten Fettalkoholpolyglycolether nach Anspruch 1 zur Herstellung von kosmetischen und/oder pharmazeutischen Zubereitungen.

7. Verwendung der verzweigten, weitgehend ungesättigten Fettalkoholpolyglycolether nach Anspruch 1 in Emulsionen für Schmierstoffanwendung.

8. Verwendung der verzweigten, weitgehend ungesättigten Fettalkoholpolyglycolether nach Anspruch 1 in Mitteln zur Bekämpfung von Moskitolarven.

## Claims

1. A process for the production of branched, substantially unsaturated fatty alcohol polyglycol ethers in which
(a) unsaturated C₁₆₋₂₂ fatty acids are dimerized in known manner,
(b) the monomer fraction accumulating in the dimerization step is removed,
(c) the branched, substantially unsaturated fatty acids present in this fraction are converted into the corresponding fatty acid methyl esters,
(d) the branched, substantially unsaturated fatty acid methyl esters are hydrogenated with the double bonds intact to form the corresponding branched, substantially unsaturated fatty alcohols which are then
(e) alkoxylated in known manner.

2. A process as claimed in claim 1, **characterized in that** the monomer fraction accumulating in the dimerization step is first subjected to fractional crystallization and the liquid phase obtained is esterified, optionally after distillation.

3. A process as claimed in claims 1 and/or 2, **characterized in that** the methyl esters and/or the fatty alcohols are subjected to distillation and/or fractional crystallization.

4. A process as claimed in at least one of claims 1 to 3, **characterized in that** on average 1 to 50 mol alkylene oxide are added onto the branched, substantially unsaturated fatty alcohols.

5. The use of the branched substantially unsaturated fatty alcohol polyglycol ethers claimed in claim 1 for the production of laundry detergents, dishwashing detergents, cleaners and softeners.

6. The use of the branched, substantially unsaturated fatty alcohol polyglycol ethers claimed in claim 1 for the production of cosmetic and/or pharmaceutical preparations.

7. The use of the branched, substantially unsaturated fatty alcohol polyglycol ethers claimed in claim 1 in emulsions for lubricant applications.

8. The use of the branched, substantially unsaturated fatty alcohol polyglycol ethers claimed in claim 1 in preparations for controlling mosquito larvae.

## Revendications

1. Procédé pour préparer des polyglycoléthers d'alcools gras ramifiés et largement insaturés, selon lequel :
(a) on dimérise de manière connue en elle même des acides gras insaturés ayant 16 à 22 atomes de carbone,
(b) on sépare la fraction monomère obtenue lors de la dimérisation,
(c) on transforme les acides gras ramifiés et largement insaturés contenus dans cette fraction en les esters méthyliques d'acides gras correspondants,
(d) on hydrogène les esters méthyliques d'acides gras ramifiés et largement insaturés en conservant les doubles liaisons pour obtenir les alcools gras ramifiés et largement insaturés correspondants, et
(e) on alcoxyle ces alcools gras de manière connue en elle-même en conservant la double liaison.

2. Procédé selon la revendication 1,
**caractérisé en ce qu'**
on soumet d'abord la fraction monomère obtenue lors de la dimérisation à une cristallisation fractionnée, puis on soumet la phase liquide ainsi obtenue à l'estérification le cas échéant après distillation.

3. Procédé selon les revendications 1 et/ou 2,
**caractérisé en ce qu'**
on soumet les esters méthyliques et/ou les alcools gras à une distillation et/ou à une cristallisation fractionnée.

4. Procédé selon au moins l'une des revendications 1 à 3,
**caractérisé en ce qu'**
on fixe en moyenne 1 à 50 moles d'oxyde d'alkylène par addition sur les alcools gras ramifiés et largement insaturés.

5. Utilisation des polyglycoléthers d'alcools gras ramifiés et largement insaturés selon la revendication 1, pour préparer des détergents, des agents de lavage, des agents de nettoyage et des agents d'avivage.

6. Utilisation des polyglycoléthers d'alcools gras ramifiés et largement insaturés selon la revendication 1, pour obtenir des préparations cosmétiques et/ou pharmaceutiques.

7. Utilisation des polyglycoléthers d'alcools gras ramifiés et largement insaturés selon la revendication 1, dans des émulsions destinées à être employées comme lubrifiants.

8. Utilisation des polyglycoléthers d'alcools gras ramifiés et largement insaturés selon la revendication 1, dans des agents destinés à combattre les larves de moustique.
